# EUROPÄISCHE PATENTSCHRIFT

(11) **EP 0 746 548 B1**
(45) Veröffentlichungstag und Bekanntmachung des Hinweises auf die Patenterteilung: **29.12.1997**
(21) Anmeldenummer: 95905642.5
(22) Anmeldetag: 21.01.1995
(51) Int. Cl.: C07D 213/89

(54) **VERFAHREN ZUR HERSTELLUNG VON 6-ARYLOXYMETHYL-1-HYDROXY-4-METHYL-2-PYRIDONEN**
METHOD OF PREPARING 6-ARYLOXYMETHYL-1-HYDROXY-4-METHYL-2-PYRIDONES
PROCEDE DE PREPARATION DE 6-ARYLOXYMETHYL-1-HYDROXY-4-METHYL-2-PYRIDONES

(30) Priorität: 23.02.1994 DE 4405722
(43) Veröffentlichungstag der Anmeldung: 11.12.1996
(73) Patentinhaber: HOECHST AKTIENGESELLSCHAFT, 65926 Frankfurt am Main (DE)
(72) Erfinder: KOCH, Peter, D-63179 Obertshausen (DE); DELPY, Klaus, D-63128 Dietzenbach (DE); SCHROD, Manfred, D-64331 Weiterstadt (DE)
(74) Vertreter: Muley, Ralf, Dr.
(86) Internationale Anmeldenummer: EP9500220
(87) Internationale Veröffentlichungsnummer: WO9523136

(56) Entgegenhaltungen:
- EP-A- 0 241 918

## Beschreibung

Die vorliegende Erfindung betrifft ein Verfahren zur Herstellung von 1-Hydroxypyridonen der allgemeinen Formel I, 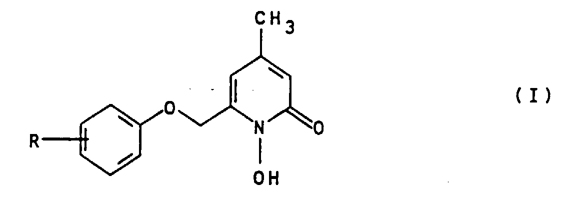 in der R für einen substituierten oder unsubstituierten Phenylrest oder Phenoxyrest steht.

Verschiedene 1-Hydroxypyridone sind wertvolle biologische Wirkstoffe, beispielsweise findet das 6-Cyclohexyl-1-hydroxy-4-methyl-2-pyridon (Ciclopirox) Verwendung als Wirkstoff in Arzneimitteln zur Behandlung von Pilzinfektionen. Auch die 1-Hydroxypyridone der allgemeinen Formel I, die in der EP-B-241 918 beschrieben sind, haben sehr gute antimykotische Eigenschaften und finden Interesse für die Verwendung in Arzneimitteln, beispielsweise das 6-((4-(4-Chlorphenoxy)phenoxy)methyl)-1-hydroxy-4-methyl-2-pyridon.

Die letzte Stufe der Synthese der Verbindungen der allgemeinen Formel I umfaßt die Umsetzung der Pyrone der allgemeinen Formel II, 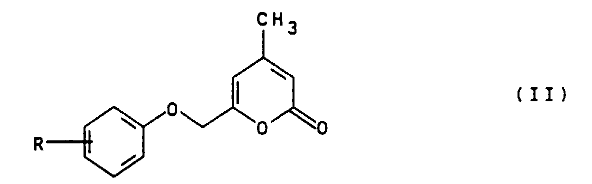 die ebenfalls in der EP-B-241 918 und der US-A-4 797 409 beschrieben sind, mit Hydroxylamin bzw. einem Hydroxylammoniumsalz in Gegenwart einer Base. Die bekannten Verfahren zur Durchführung dieser Umsetzung haben aber Nachteile, die einer Übertragung in den technischen Maßstab entgegenstehen.

In der EP-B-241 918 sind Verfahren beschrieben, bei denen Pyrone der allgemeinen Formel II mit Hydroxylammoniumchlorid in 2-Aminopyridin oder mit Hydroxylammoniumsulfat in Gegenwart von Natriumcarbonat in Toluol umgesetzt werden. Bei der Aufarbeitung wird aber Methylenchlorid eingesetzt. Der Einsatz dieses leichtflüchtigen chlorierten Kohlenwasserstoffs in einem technischen Verfahren verbietet sich aus ökologischen Gründen bzw. würde außerordentliche Aufwendungen für die Behandlung der Abluft und des Abwassers erfordern. Zudem ist die für die dort beschriebene Herstellung von 6-((4-(4-Chlorphenoxy)phenoxy)-methyl)-1-hydroxy-4-methyl-2-pyridon angegebene Ausbeute von 45 % für ein technisches Verfahren unbefriedigend. Auch andere in der Literatur (s. z.B. DE-B-22 14 608 oder Arzneim.-Forsch./Drug Res. 31(II), S. 1311 - 1316 (1981)) beschriebene Synthesen von 1-Hydroxy-2-pyridonen haben solche technischen Nachteile oder erweisen sich für die Verbindungen der allgemeinen Formel I als ungeeignet.

Das in der US-A-4 916 228 beschriebene Verfahren zur Herstellung von 1-Hydroxy-2-pyridonen kommt ohne ökologisch bedenkliche Hilfsstoffe aus. Die Isolierung erfolgt aber in Form der Ethanolamin-Salze, zu deren Herstellung das 1-Hydroxypyridon in Lösung vorliegen muß. Beschrieben ist die Anwendung dieses Verfahrens für die Herstellung von 1-Hydroxy-4-methyl-2-pyridonen, die in der 6-Position einen Cyclohexylrest oder einen 2,4,4-Trimethylpentylrest tragen. Aufgrund der anderen Löslichkeitseigenschaften der Verbindungen der allgemeinen Formel I, die in der 6-Position den aromatischen Biphenylyloxymethylrest oder den Phenoxyphenoxymethylrest tragen, erweist sich für diese das Verfahren der US-A-4 916 228 aber als nicht anwendbar. Zudem wäre die Überführung der Ethanolamin-Salze in die freien 1-Hydroxypyridone mit einem zusätzlichen Arbeitsschritt verbunden.

Aufgabe der vorliegenden Erfindung ist es, ein technisch einfaches, in ökologischer und ökonomischer Hinsicht günstigeres Verfahren zur Herstellung von 1-Hydroxypyridonen der allgemeinen Formel I bereitzustellen. Überraschend wurde gefunden, daß diese Aufgabe gelöst wird, indem zunächst in an sich bekannter Weise das Pyron der allgemeinen Formel II mit Hydroxylamin oder einem Hydroxylammoniumsalz in Gegenwart einer Base umgesetzt wird und dann die Isolierung des Produkts aus einem Gemisch aus Wasser und wassermischbaren organischen Lösungsmitteln im sauren Milieu erfolgt. Dies ist umso erstaunlicher, als aus der Literatur (Arzneim.-Forsch./Drug Res. 31(II), 1311 - 1316 (1981)) bekannt ist, daß die Umsetzung von Pyronen mit Hydroxylamin nicht einheitlich verläuft, sondern als Nebenprodukte Isoxazolinessigsäuren liefert, die beim Ansäuern der Reaktionsmischung zumindest aus einem wäßrigen System ausfallen sollten, und als bei der Kristallisation des Produkts aus einem organische Lösungsmittel enthaltenden System eher zu erwarten wäre, daß größere Mengen 1-Hydroxypyridon der allgemeinen Formel I in der Mutterlauge gelöst verbleiben und die Ausbeute vermindert wird. Stattdessen ist sie höher.

Gegenstand der vorliegenden Erfindung ist somit ein Verfahren zur Herstellung von 1-Hydroxpyridonen der allgemeinen Formel I, 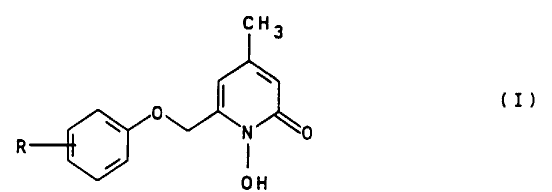 in der R für einen Phenylrest oder einen Phenoxyrest steht, wobei der Phenylrest und der Phenoxyrest auch durch einen oder mehrere gleiche oder verschiedene Substituenten aus der Reihe (C₁-C₄)-Alkyl, Halogen, Trifluormethyl, (C₁-C₄)-Alkoxy und Trifluormethoxy substituiert sein kann, durch Umsetzung von Pyronen der allgemeinen Formel II, 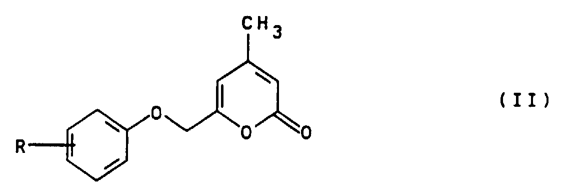 in der R wie für die Formel I angegeben definiert ist, mit Hydroxylamin oder einem Hydroxylammoniumsalz in Gegenwart einer Base, dadurch gekennzeichnet, daß die Isolierung des Produkts aus einem Gemisch aus Wasser und einem oder mehreren wassermischbaren organischen Lösungsmitteln erfolgt und bei der Aufarbeitung angesäuert wird.

(C₁-C₄)-Alkyl-Substituenten können geradkettig oder verzweigt sein, entsprechendes gilt für (C₁-C₄)-Alkoxy-Substituenten. Beispiele für (C₁-C₄)-Alkylgruppen, die als solche oder in (C₁-C₄)-Alkoxygruppen auftreten können, sind Methyl, Ethyl, Propyl, Isopropyl, Butyl, Isobutyl, s-Butyl und tert.-Butyl. Bevorzugte Alkylgruppen sind Methyl und tert.-Butyl. Bevorzugte Alkoxygruppen sind Methoxy und tert.-Butoxy.

Beispiele für Halogen sind Fluor, Chlor, Brom und Iod. Bevorzugt sind Fluor, Chlor und Brom, insbesondere Chlor.

Der Rest R in der allgemeinen Formel I kann in der 2-Position, der 3-Position oder der 4-Position stehen. Bevorzugt steht er in der 3-Position oder der 4-Position, besonders bevorzugt in der 4-Position. Der Phenylrest und der Phenoxyrest, für die R steht, kann unsubstituiert oder einfach oder mehrfach substituiert sein. Bevorzugt ist er unsubstituiert oder einfach, zweifach oder dreifach substituiert. Besonders bevorzugt ist er unsubstituiert oder einfach oder zweifach substituiert. Die Substituenten können in beliebigen Positionen stehen.

Bevorzugt werden nach dem erfindungsgemäßen Verfahren 1-Hydroxypyridone der allgemeinen Formel I hergestellt, in der R für einen Phenoxyrest steht, der auch durch einen oder zwei gleiche oder verschiedene Substituenten aus der Reihe Fluor, Chlor, Brom, Trifluormethyl und Trifluormethoxy substituiert sein kann.

Besonders bevorzugt werden nach dem erfindungsgemäßen Verfahren 1-Hydroxypyridone der allgemeinen Formel I hergestellt, in der R für einen Phenoxyrest steht, der durch einen oder zwei Substituenten aus der Reihe Fluor, Chlor und Brom substituiert ist. Darüber hinaus bevorzugt wird das 1-Hydroxypyridon der allgemeinen Formel I hergestellt, in dem R für einen chlorsubstituierten Phenoxyrest steht, insbesondere für einen 4-ständigen 4-Chlorphenoxyrest. Weitere Verbindungen, die nach dem erfindungsgemäßen Verfahren hergestellt werden können, sind diejenigen der allgemeinen Formel I, in der R für einen 4-ständigen Phenylrest oder für einen 4-ständigen 4-Trifluormethylphenoxyrest steht.

Die Umsetzung des Pyrons der allgemeinen Formel II mit Hydroxylamin oder einem Hydroxylammoniumsalz in Gegenwart einer Base kann mit oder ohne Lösungsmittel durchgeführt werden. Bevorzugt wird sie in einem Lösungsmittel durchgeführt. Als Lösungsmittel können beispielsweise Ester, wie Ethylacetat oder Butylacetat, Ether, wie Dimethoxyethan, Diethylenglykoldimethylether, Dioxan oder Tetrahydrofuran, Alkohole, wie Methanol, Ethanol, Propanol, Butanol oder Ethylenglykolmonomethyl-, -ethyl- oder -butylether, Wasser, Amide, wie Dimethylformamid oder N-Methylpyrrolidon, oder beispielsweise Dimethylsulfoxid eingesetzt werden. Bevorzugt als Lösungsmittel sind aliphatische oder aromatische Kohlenwasserstoffe, wie z.B. Hexan, Heptan, Benzin-Fraktionen, Cyclohexan, Methylcyclohexan, Benzol, Toluol, Xylol, Chlorbenzol oder Dichlorbenzol. Besonders bevorzugt sind Kohlenwasserstoffe aus der Reihe Heptan, Toluol, Xylol, Chlorbenzol und Dichlorbenzol. Lösungsmittel können sowohl allein als auch im Gemisch mit einem oder mehreren anderen Lösungsmitteln eingesetzt werden. Ganz besonders bevorzugt erfolgt die Umsetzung in Toluol.

Bevorzugt wird die Umwandlung des Pyrons der allgemeinen Formel II in das 1-Hydroxypyridon der allgemeinen Formel I durchgeführt, indem das Pyron der allgemeinen Formel II und gegebenenfalls das Lösungsmittel oder ein Teil des Lösungsmittels bei der gewünschten Reaktionstemperatur vorgelegt wird und dann das Hydroxylamin oder das Hydroxylammoniumsalz und die Base zudosiert wird. Hydroxylamin liegt handelsüblich in Form von Salzen, z.B. als Salz der Salz-, Schwefel-, Phosphor- oder Essigsäure, vor. Diese Salze können als solche in fester oder gelöster oder suspendierter Form zudosiert werden, oder es kann in einem separaten Schritt aus dem Salz mit einer Base das Hydroxylamin freigesetzt werden und als solches, bevorzugt in Form einer gegebenenfalls filtrierten Lösung, zudosiert werden. Insbesondere wenn ein Hydroxylammoniumsalz in fester Form eingesetzt wird, erfolgt die Zugabe zum Pyron der allgemeinen Formel II bevorzugt in mehreren Portionen, besonders bevorzugt in Abständen, die dem Reaktionsverlauf angepaßt sind. Das Hydroxylamin oder Hydroxylammoniumsalz wird bevorzugt in einer Menge von 1 bis 10 Mol pro Mol des Pyrons der allgemeinen Formel II eingesetzt, besonders bevorzugt in einer Menge von 1,3 bis 3 Mol pro Mol des Pyrons. Bevorzugt eingesetzt wird Hydroxylammoniumchlorid oder Hydroxylammoniumsulfat.

Als Base für die Freisetzung des Hydroxylamins aus seinem Salz kommen anorganische und organische Basen in Betracht, beispielsweise sekundäre und tertiäre Amine, z.B. Diisopropylamin, Triethylamin, Tributylamin, Triethanolamin, oder Stickstoffheterocyclen wie Pyridin, Chinolin oder Imidazol. Bevorzugt werden anorganische Basen eingesetzt, besonders bevorzugt Oxide, Hydroxide, Carbonate oder Hydrogencarbonate der Alkali- oder Erdalkalimetalle, aber auch z.B. Acetate oder basisch reagierende Phosphate dieser Metalle. Beispiele für Alkali- und Erdalkalimetalle sind insbesondere Lithium, Natrium, Kalium, Caesium, Magnesium, Calcium und Barium, wobei Natrium und Kalium bevorzugt sind. Ganz besonders bevorzugt werden in dem erfindungsgemäßen Verfahren als Base Natriumhydroxid, Natriumcarbonat, Kaliumhydroxid oder Kaliumcarbonat eingesetzt. Basen können sowohl allein als auch im Gemisch mit einer oder mehreren anderen Basen eingesetzt werden. Die Base wird üblicherweise in einer zur Menge des eingesetzten Hydroxylammoniumsalzes äquivalenten Menge eingesetzt, es kann aber auch ein Überschuß oder ein geringer Unterschuß an Base günstig sein. Die Base kann in fester oder gelöster oder suspendierter Form eingesetzt werden. Wird in der bevorzugten Ausführungsform des erfindungsgemäßen Verfahrens ein Hydroxylammoniumsalz als solches zum Pyron der allgemeinen Formel II zugegeben, so erfolgt die Zugabe der Base bevorzugt parallel dazu. Wird das Hydroxylammoniumsalz in mehreren Portionen zudosiert, so wird bevorzugt auch die Base in mehreren Portionen zudosiert. Nach Beendigung der Zugabe des Hydroxylamins oder des Hydroxylammoniumsalzes und der Base wird das Reaktionsgemisch bei der gewünschten Temperatur nachgerührt, bis der gewünschte Umsetzungsgrad erreicht ist. Im allgemeinen sind zur vollständigen Umsetzung etwa 4 bis 14 Stunden erforderlich.

Die Umsetzung des Pyrons der allgemeinen Formel II mit Hydroxylamin oder einem Hydroxylammoniumsalz wird zweckmäßigerweise bei Temperaturen unter 120°C durchgeführt. Bevorzugt wird sie bei 20 bis 115°C, besonders bevorzugt bei 20 bis 100°C, darüber hinaus bevorzugt bei 50 bis 100°C durchgeführt. Die Temperatur kann während der Durchführung der Umsetzung verändert werden, z.B. beim Nachrühren zur Vervollständigung der Reaktion erhöht werden.

Vorteilhaft ist die einfache Aufarbeitung beim Arbeiten nach dem erfindungsgemäßen Verfahren. Wurde bei der Überführung des Pyrons der allgemeinen Formel II in das 1-Hydroxypyridon ohne Lösungsmittel gearbeitet, so werden nach beendeter Umsetzung Wasser und ein oder mehrere wassermischbare Lösungsmittel zugegeben. Wurde die Umsetzung des Pyrons mit Hydroxylamin oder einem Hydroxylammoniumsalz in einem Lösungsmittel durchgeführt, das zur Produktisolierung entfernt werden soll, so kann die Aufarbeitung des Reaktionsgemisches derart erfolgen, daß zunächst ein Teil oder die Gesamtmenge dieses Lösungsmittels im Vakuum oder bei Normaldruck abdestilliert wird und dann der Rückstand mit Wasser und einem oder mehreren wassermischbaren organischen Lösungsmitteln versetzt wird, oder es kann zum Reaktionsgemisch erst Wasser und/oder ein wassermischbares organisches Lösungsmittel zugegeben werden, dann das zu entfernende Lösungsmittel abdestilliert werden, gegebenenfalls als Azeotrop, und dann der Rückstand anschließend noch mit einem oder mehreren wassermischbaren organischen Lösungsmittel und/oder Wasser versetzt werden. Bevorzugt wird zum Reaktionsgemisch erst Wasser zugegeben, dann das zu entfernende Lösungsmittel abdestilliert, gegebenenfalls als Azeotrop mit Wasser und besonders günstig am Phasenscheider, und dann der wäßrige Rückstand mit einem oder mehreren wassermischbaren organischen Lösungsmitteln versetzt. Je nach den Löslichkeitsverhältnissen der Produkte und Salze in den angewandten Lösungsmitteln kann es auch günstig sein, bei der Aufarbeitung einen Filtrationsschnitt oder eine Phasentrennung einzuschieben. Abdestilliertes Lösungsmittel kann für einen Folgeansatz der Umsetzung des Pyrons der allgemeinen Formel II mit Hydroxylamin oder einem Hydroxylammoniumsalz verwendet werden. In jedem Falle wird bei der Aufarbeitung ein Zustand hergestellt, bei dem das Produkt der Umsetzung des Pyrons der allgemeinen Formel II mit Hydroxylamin oder einem Hydroxylammoniumsalz in einem Gemisch aus Wasser und einem oder mehreren, bevorzugt einem, wassermischbaren organischen Lösungsmitteln vorliegt.

Als wassermischbare organische Lösungsmittel werden bevorzugt solche aus der Reihe der wassermischbaren Alkohole, Ether, Carbonsäuren und Ketone eingesetzt. Beispiele für geeignete Lösungsmittel sind Methanol, Ethanol, Propanol, Isopropanol, Ethylenglykol, Di- und Triethylenglykol, Propylenglykol, Tetrahydrofuran, Dioxan, Ethylenglykolmono- und -dimethylether, Diethylenglykolmono- und -dimethylether, Ameisensäure, Essigsäure und Aceton. In Frage kommen aber auch Amide, wie Formamid, Dimethylformamid oder N-Methylpyrrolidon, oder beispielsweise Acetonitril oder Dimethylsulfoxid. Besonders bevorzugt werden wassermischbare organische Lösungsmittel aus der Reihe Methanol, Ethanol, Isopropanol, Ethylenglykoldimethylether, Essigsäure und Aceton eingesetzt. Darüber hinaus bevorzugt wird als wassermischbares organisches Lösungsmittel Methanol eingesetzt. Die Mengenanteile der Komponenten des Gemisches aus Wasser und einem oder mehreren wassermischbaren organischen Lösungsmitteln richten sich nach den Löslichkeitseigenschaften der jeweiligen Substanzen.

Das Ansäuern des bei der Umsetzung des Pyrons der allgemeinen Formel II mit Hydroxylamin oder einem Hydroxylammoniumsalz erhaltenen Reaktionsgemisches kann vor oder nach Zugabe von Wasser und wassermischbaren organischen Lösungsmitteln erfolgen. Wurde das Pyron in einem Lösungsmittel umgesetzt und soll dieses zur Produktisolierung entfernt werden, indem Wasser zugegeben wird und dann dieses Lösungsmittel abdestilliert wird, so kann das Ansäuern vor oder nach der Wasserzugabe, vor oder nach dem Destillieren oder auch erst nach der Zugabe eines oder mehrerer wassermischbarer organischer Lösungsmittel erfolgen. Bevorzugt erfolgt das Ansäuern, wenn das Produkt der Umsetzung des Pyrons bereits in dem für die Isolierung vorgesehenen Gemisch aus Wasser und einem oder mehreren wassermischbaren organischen Lösungsmitteln vorliegt. Bevorzugt wird bei der Aufarbeitung ein pH-Wert zwischen 0 und 6, besonders bevorzugt zwischen 1 und 5, ganz besonders bevorzugt zwischen 2 und 4 eingestellt. Zum Ansäuern wird bevorzugt Schwefelsäure, Salzsäure, Phosphorsäure oder Essigsäure verwendet. Geeignete Säuren sind aber beispielsweise auch Salpetersäure, Ameisensäure, Trifluoressigsäure oder Methansulfonsäure. Es können auch Gemische von Säuren verwendet werden. Die Säuren können in reiner Form oder in Form von Lösungen beliebiger, insbesondere handelsüblicher, Konzentrationen eingesetzt werden.

Das aus dem sauren Gemisch aus Wasser und einem oder mehreren wassermischbaren organischen Lösungsmitteln auskristallisierende 1-Hydroxypyridon der allgemeinen Formel I kann in üblicher Weise durch einfaches Filtrieren oder Zentrifugieren isoliert werden und wird üblicherweise mit Wasser und/oder einem Gemisch aus Wasser und dem oder den eingesetzten wassermischbaren organischen Lösungsmitteln und/oder mit dem oder den eingesetzten organischen Lösungsmittel gewaschen und getrocknet. Im Filtrat enthaltene wassermischbare organische Lösungsmittel können durch Destillation wiedergewonnen und im Folgeansatz eingesetzt werden.

Nach dem erfindungsgemäßen Verfahren hergestellte 1-Hydroxypyridone der allgemeinen Formel I fallen in einer besonders gut filtrierbaren Form an. Sie haben eine hohe Reinheit, so daß, wenn für bestimmte Verwendungszwecke eine weitere Reinigung durch Umkristallisation erforderlich ist, diese nur noch mit geringen Verlusten verbunden ist. Nach dem erfindungsgemäßen Verfahren werden die 1-Hydroxypyridone der allgemeinen Formel I in deutlich höheren Ausbeuten als nach den bekannten Verfahren erhalten, im Falle des 6-((4-(4-Chlorphenoxy)phenoxy)methyl)-1-hydroxy-4-methyl-2-pyridons z.B. in 54 bis 66 % gegenüber 45 % nach dem Verfahren der EP-B-241 918. Daß diese Vorteile damit verknüpft sind, daß - erfindungsgemäß - sowohl ein wassermischbares organisches Lösungsmittel verwendet wird als auch angesäuert wird, wird deutlich, wenn eine dieser Bedingungen nicht erfüllt ist. Wird 6-((4-(4-Chlorphenoxy)phenoxy)methyl-1-hydroxy-4-methyl-2-pyridon nach dem erfindungsgemäßen Verfahren hergestellt, dabei aber kein organisches Lösungsmittel zugesetzt, sondern rein wäßrig gearbeitet, so enthält das ausgefallene Produkt die als Nebenprodukt entstandene Isoxazolinessigsäure, und die Ausbeute beträgt nur ca. 45 %. Auch wenn ein wassermischbares organisches Lösungsmittel zugesetzt wird, aber nicht sauer gestellt wird, liegt die Ausbeute nur bei 45 %.

Die nachfolgenden Beispiele erläutern die Erfindung.

### Beispiele 1 bis 13: Herstellung von 6-((4-(4-Chlorphenoxy)phenoxy)methyl)-1-hydroxy-4-methyl-2-pyridon

### Beispiel 1

In einem 2 l-Dreihalskolben mit KPG-Rührer und Thermometer werden 369,6 g (1,08 Mol) 6-((4-(4-Chlorphenoxy)phenoxy)methyl)-4-methyl-2-pyron in 600 ml Toluol vorgelegt und auf 90°C erwärmt. Bei dieser Temperatur werden in Abstand von je 15 min in je 10 Portionen insgesamt 114,6 g (1,08 Mol) Natriumcarbonat und 150 g (2,16 Mol) Hydroxylammoniumchlorid gegeben. Nach 10 h Nachrühren bei 90°C gibt man 900 ml Trinkwasser dazu und destilliert Toluol am Phasenscheider ab. Den wäßrigen Rückstand gibt man in 3 l Methanol, stellt mit 37 %iger Schwefelsäure auf pH 3, bringt den Ansatz auf Raumtemperatur und saugt ab. Es wird mit 300 ml Methanol und 300 ml Trinkwasser gewaschen. Die feuchte Paste wird zur Entfernung restlicher Salze zweimal mit je 900 ml Trinkwasser bei 60°C gerührt, abgesaugt und mit je 300 ml Wasser gewaschen. Die Hälfte des feuchten Produkts wird getrocknet.
Ausbeute 107,7 g, entsprechend 55,8 %. Fp. 168°C.

Die andere Hälfte des feuchten Produkts wird in 450 ml Toluol gegeben, Wasser am Phasenscheider abdestilliert und die klare Lösung auf 5°C gekühlt. Das auskristallisierte Produkt wird abgesaugt, mit kaltem Toluol gewaschen und getrocknet.
Ausbeute 104,7 g, entsprechend 54,3 %. Fp. 168°C.

### Beispiel 2

Man verfährt wie in Beispiel 1, setzt jedoch statt 1,08 Mol Natriumcarbonat 2,16 Mol NaOH-Pulver ein. Ausbeute 221,5 g, entsprechend 57,4 %. Fp. 167°C.

### Beispiele 3 bis 6

Verfährt man wie in Beispiel 1, setzt jedoch statt Natriumcarbonat äquivalente Mengen Natriumhydrogencarbonat, Kaliumhydroxid, Kaliumhydrogencarbonat oder Kaliumcarbonat ein, so erhält man vergleichbare Ergebnisse.

### Beispiele 7 bis 10

Verfährt man wie in Beispiel 1, setzt jedoch statt Methanol Ethanol, Ethylenglykoldimethylether oder Aceton ein, erhält man vergleichbare Ergebnisse.

### Beispiele 11 und 12

Verfährt man wie in Beispiel 1, setzt jedoch statt Schwefelsäure Salzsäure oder Phosphorsäure ein, so werden vergleichbare Resultate erhalten.

### Beispiel 13

In einem 2 l-Dreihalskolben mit KPG-Rührer, Thermometer und Rückflußkühler werden 342,8 g (1 Mol) 6-((4-(4-Chlorphenoxy)phenoxy)methyl)-4-methyl-2-pyron in 500 ml Toluol vorgelegt und auf 90°C erwärmt. Innerhalb von neun Stunden werden im Abstand von je einer Stunde 76 g (1,9 Mol) natriumhydroxid-Plätzchen und 132,1 g (1,9 Mol) Hydroxylammoniumchlorid in jeweils zehn Portionen zugegeben. Nach vier Stunden Nach-rühren bei 90°C werden 500 ml Wasser zugesetzt und es wird bei Normaldruck Toluol am Phasenscheider abdestilliert. Die wäßrige Suspension wird mit 1,7 1 Methanol versetzt und mit 37 %iger Schwefelsäure auf pH 3 gestellt. Der Ansatz wird auf Raumtemperatur gebracht und der Niederschlag abgesaugt und mit 300 ml Methanol und 300 ml Wasser gewaschen. Zur Entfernung restlicher Salze wird die feuchte Paste noch zweimal mit je 900 ml Trinkwasser bei 60°C gerührt, abgesaugt und mit 300 ml Trinkwasser gewaschen. Das feuchte Produkt wird in 800 ml Toluol eingetragen, am Phasenscheider wasserfrei destilliert und die klare Lösung auf 5°C abgekühlt. Das auskristallisierte Produkt wird abgesaugt, mit kaltem Toluol gewaschen und getrocknet.
Ausbeute 235 g, entsprechend 65,6 %; Fp. 167°C.

### Beispiel 14

### Herstellung von 6-((4-Phenylphenoxy)methyl)-1-hydroxy-4-methyl-2-pyridon

58,4 g (0,2 Mol) 6-((4-Phenylphenoxy)methyl)-4-methyl-2-pyron werden in 200 ml Toluol vorgelegt und auf 90°C erwärmt. Im Abstand von je einer Stunde werden in jeweils zehn Portionen insgesamt 15,2 g (0,38 Mol) NaOH-Plätzchen und 26,4 g (0,38 Mol) Hydroxylammoniumchlorid eingetragen. Nach drei Stunden Nachrühren bei 90°C werden 200 ml Trinkwasser zugesetzt und es wird Toluol am Phasenscheider abdestilliert. Der Ansatz wird mit 500 ml Methanol versetzt und mit 37 %iger Schwefelsäure auf pH 3 gestellt. Nach Abkühlen auf Raumtemperatur wird der Niederschlag abgesaugt und mit zweimal je 20 ml Methanol und zweimal je 20 ml Trinkwasser gewaschen. Das feuchte Produkt wird zweimal mit je 60 ml Trinkwasser bei 60°C ausgerührt und jeweils abgesaugt und mit je 20 ml warmem Trinkwasser gewaschen und getrocknet. Es wird aus 215 ml Toluol umkristallisiert, getrocknet und mit 265 ml Methanol unter Rückfluß ausgerührt, abgesaugt, mit 40 ml Methanol gewaschen und getrocknet. Ausbeute 31,6 g, entsprechend 51,5 %; Fp. 189-193°C (Lit. EP-B-241918:184°C).

### Beispiel 15

### Herstellung von 6-((4-(4-Trifluormethylphenoxy)phenoxy) methyl)-1-hydroxy-4-methyl-2-pyridon

26,5 g (70 mMol) 6-((4-(4-Trifluormethylphenoxy)phenoxy) methyl)-4-methyl-2-pyron werden in 140 ml Toluol auf 90°C erwärmt und in elf Portionen im Abstand von jeweils einer Stunde mit insgesamt 16,4 g (155 mMol) Natriumcarbonat und 10,9 g (157 mMol) Hydroxylammoniumchlorid versetzt. Nach 20 Stunden Nachrühren bei 90°C werden 140 ml Trinkwasser zugegeben und es wird Toluol am Phasenscheider abdestilliert. Zur wäßrigen Suspension gibt man 560 ml Methanol und stellt mit 37 %iger Schwefelsäure auf pH 3. Nach Abkühlen auf Raumtemperatur wird abgesaugt, mit zweimal je 56 ml Methanol und insgesamt 1130 ml Trinkwasser von 60°C in Portionen gewaschen und getrocknet.

Es wird aus 35 ml Toluol umkristallisiert.
Ausbeute 8,6 g, entsprechend 31,2 %; Fp. 156-157°C (Lit. EP-B-241918:149°C).

## Patentansprüche

1. Verfahren zur Herstellung von 1-Hydroxypyridonen der allgemeinen Formel I, 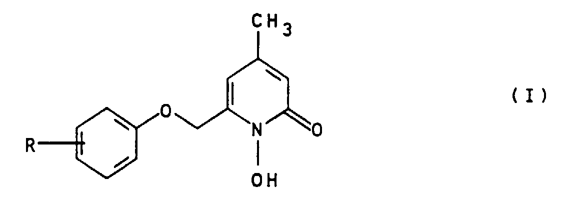 in der R für einen Phenylrest oder einen Phenoxyrest steht, wobei der Phenylrest und der Phenoxyrest auch durch einen oder mehrere gleiche oder verschiedene Substituenten aus der Reihe (C₁-C₄)-Alkyl, Halogen, Trifluormethyl, (C₁-C₄)-Alkoxy und Trifluormethoxy substituiert sein kann, durch Umsetzung von Pyronen der allgemeinen Formel II, 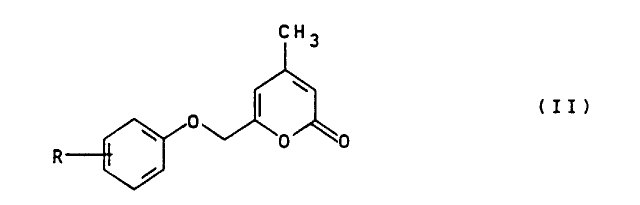 in der R wie für die Formel I angegeben definiert ist, mit Hydroxylamin oder einem Hydroxylammoniumsalz in Gegenwart einer Base, dadurch gekennzeichnet, daß die Isolierung des Produkts aus einem Gemisch aus Wasser und einem oder mehreren wassermischbaren organischen Lösungsmitteln erfolgt und bei der Aufarbeitung angesäuert wird.

2. Verfahren gemäß Anspruch 1, dadurch gekennzeichnet, daß R für einen Phenoxyrest steht, der auch durch einen oder zwei gleiche oder verschiedene Substituenten aus der Reihe Fluor, Chlor, Brom, Trifluormethyl und Trifluormethoxy substituiert sein kann.

3. Verfahren gemäß Anspruch 1 und/oder 2, dadurch gekennzeichnet, daß R für einen 4-ständigen 4-Chlorphenoxyrest steht.

4. Verfahren gemäß einem oder mehreren der Ansprüche 1 bis 3, dadurch gekennzeichnet, daß die Umsetzung des Pyrons der allgemeinen Formel II mit Hydroxylamin oder einem Hydroxylammoniumsalz in einem aliphatischen oder aromatischen Kohlenwasserstoff, bevorzugt in einem Kohlenwasserstoff aus der Reihe Heptan, Toluol, Xylol, Chlorbenzol und Dichlorbenzol, besonders bevorzugt in Toluol, erfolgt.

5. Verfahren gemäß einem oder mehreren der Ansprüche 1 bis 4, dadurch gekennzeichnet, daß als Base ein Oxid, Hydroxid, Carbonat oder Hydrogencarbonat der Alkali- oder Erdalkalimetalle oder ein Gemisch dieser Verbindungen, bevorzugt Natriumhydroxid, Natriumcarbonat, Kaliumhydroxid oder Kaliumcarbonat oder ein Gemisch dieser Verbindungen, eingesetzt wird.

6. Verfahren gemäß einem oder mehreren der Ansprüche 1 bis 5, dadurch gekennzeichnet, daß die Umsetzung des Pyrons der allgemeinen Formel II mit Hydroxylamin oder einem Hydroxylammoniumsalz bei 20 bis 115°C, bevorzugt bei 20 bis 100°C, besonders bevorzugt bei 50 bis 100°C, durchgeführt wird.

7. Verfahren gemäß einem oder mehreren der Ansprüche 1 bis 6, dadurch gekennzeichnet, daß das oder die wassermischbaren organischen Lösungsmittel aus der Reihe der wassermischbaren Alkohole, Ether, Carbonsäuren und Ketone, bevorzugt aus der Reihe Methanol, Ethanol, Isopropanol, Ethylenglykoldimethylether, Essigsäure und Aceton, ausgewählt werden.

8. Verfahren gemäß einem oder mehreren der Ansprüche 1 bis 7, dadurch gekennzeichnet, daß als wassermischbares organisches Lösungsmittel Methanol eingesetzt wird.

9. Verfahren gemäß einem oder mehreren der Ansprüche 1 bis 8, dadurch gekennzeichnet, daß bei der Aufarbeitung ein pH-Wert zwischen 0 und 6, bevorzugt zwischen 1 und 5, besonders bevorzugt zwischen 2 und 4, eingestellt wird.

10. Verfahren gemäß einem oder mehreren der Ansprüche 1 bis 9, dadurch gekennzeichnet, daß zum Ansäuern Schwefelsäure, Salzsäure, Phosphorsäure oder Essigsäure verwendet wird.

## Claims

1. A process for the preparation of 1-hydroxypyridones of the formula I 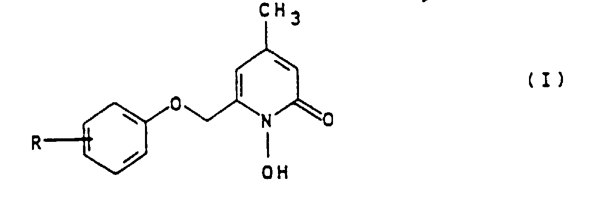 in which R is a phenyl radical or a phenoxy radical, where the phenyl radical and the phenoxy radical can also be substituted by one or more identical or different substituents selected from the group consisting of (C₁-C₄)-alkyl, halogen, trifluoromethyl, (C₁-C₄)-alkoxy and trifluoromethoxy, by reaction of pyrones of the formula II 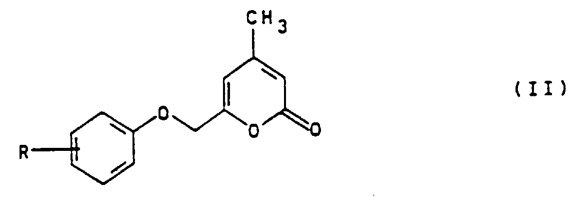 in which R is defined as indicated for the formula I, with hydroxylamine or a hydroxylammonium salt in the presence of a base, which comprises carrying out the isolation of the product from a mixture of water and one or more water-miscible organic solvents and acidifying during the work-up.

2. The process as claimed in claim 1, wherein R is a phenoxy radical which can also be substituted by one or two identical or different substituents selected from the group consisting of fluorine, chlorine, bromine, trifluoromethyl and trifluoromethoxy.

3. The process as claimed in claim 1 and/or 2, wherein R is a 4-chlorophenoxy radical in the 4-position.

4. The process as claimed in one or more of claims 1 to 3, wherein the reaction of the pyrone of the formula II with hydroxylamine or a hydroxylammonium salt is carried out in an aliphatic or aromatic hydrocarbon, preferably in a hydrocarbon selected from the group consisting of heptane, toluene, xylene, chlorobenzene and dichlorobenzene, particularly preferably in toluene.

5. The process as claimed in one or more of claims 1 to 4, wherein, as base, an oxide, hydroxide, carbonate or hydrogen carbonate of the alkali metals or alkaline earth metals or a mixture of these compounds is employed, preferably sodium hydroxide, sodium carbonate, potassium hydroxide or potassium carbonate or a mixture of these compounds.

6. The process as claimed in one or more of claims 1 to 5, wherein the reaction of the pyrone of the formula II with hydroxylamine or a hydroxylammonium salt is carried out at 20 to 115°C, preferably at 20 to 100°C, particularly preferably at 50 to 100°C.

7. The process as claimed in one or more of claims 1 to 6, wherein the water-miscible organic solvent(s) are selected from the group consisting of the water-miscible alcohols, ethers, carboxylic acids and ketones, preferably from the group consisting of methanol, ethanol, isopropanol, ethylene glycol dimethyl ether, acetic acid and acetone.

8. The process as claimed in one or more of claims 1 to 7, wherein, as water-miscible organic solvent, methanol is employed.

9. The process as claimed in one or more of claims 1 to 8, wherein during the work-up a pH of between 0 and 6, preferably between 1 and 5, particularly preferably between 2 and 4, is set.

10. The process as claimed in one or more of claims 1 to 9, wherein, for acidification, sulfuric acid, hydrochloric acid, phosphoric acid or acetic acid is used.

## Revendications

1. Procédé de préparation de 1-hydroxypyridones de formule générale I, 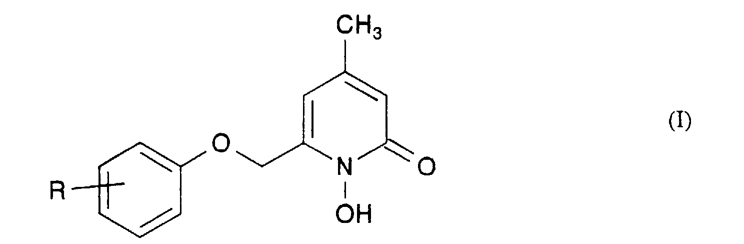 dans laquelle R représente un groupe phényle ou un groupe phénoxy, le groupe phényle et le groupe phénoxy pouvant être également substitués par un ou plusieurs substituants identiques ou différents choisis parmi un atome d'halogène et les groupes alkyle (C₁-C₄), trifluorométhyle, alcoxy (C₁-C₄) et trifluorométhoxy, par réaction de pyrones de formule générale II, 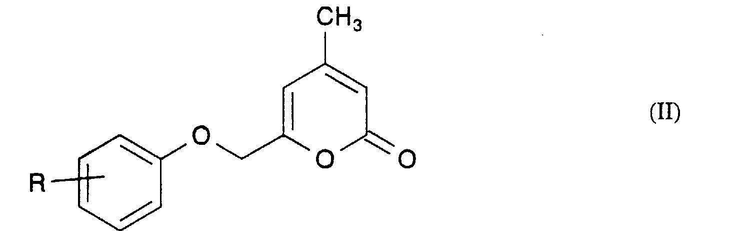 dans laquelle R est défini comme pour la formule I, avec de l'hydroxylamine ou un sel hydroxylammonium en présence d'une base, caractérisé en ce que le produit est isolé à partir d'un mélange d'eau et d'un ou de plusieurs solvants organiques miscibles à l'eau et que le traitement passe par une étape d'acidification.

2. Procédé selon la revendication 1, caractérisé en ce que R représente un groupe phénoxy qui peut être substitué par un ou deux substituants identiques ou différents choisis parmi un atome de fluor, de chlore, de brome et les groupes trifluorométhyle et trifluorométhoxy.

3. Procédé selon les revendications 1 et/ou 2, caractérisé en ce que R représente un groupe 4-chlorophénoxy en position 4.

4. Procédé selon une ou plusieurs des revendications 1 à 3, caractérisé en ce que la réaction de la pyrone de formule générale II avec l'hydroxylamine ou un sel hydroxylammonium a lieu dans un hydrocarbure aliphatique ou aromatique, de préférence dans un hydrocarbure choisi parmi l'heptane, le toluène, le xylène, de chlorobenzène et le dichlorobenzène, en particulier dans le toluène.

5. Procédé selon une ou plusieurs des revendications 1 à 4, caractérisé en ce que la base utilisée est un oxyde, un hydroxyde, un carbonate ou un hydrogénocarbonate de métaux alcalins ou alcalino-terreux ou un mélange de ces composés, de préférence l'hydroxyde de sodium, le carbonate de sodium, l'hydroxyde de potassium ou le carbonate de potassium ou un mélange de ces composés.

6. Procédé selon une ou plusieurs des revendications 1 à 5, caractérisé en ce que la réaction de la pyrpne de formule générale II avec l'hydroxylamine ou un sel hydroxylammonium est conduite de 20 à 115°C, de préférence de 20 à 100 °C, en particulier de 50 à 100 °C.

7. Procédé selon une ou plusieurs des revendications 1 à 6, caractérisé en ce que le ou les solvants organiques miscibles à l'eau sont choisis parmi les alcools, les éthers, les acides carboxyliques et les cétones miscibles à l'eau, de préférence parmi le méthanol, l'éthanol, l'isopropanol, le diméthyléther d'éthylène glycol, l'acide acétique et l'acétone.

8. Procédé selon une ou plusieurs des revendications 1 à 7, caractérisé en ce que le solvant organique miscible à l'eau utilisé est le méthanol.

9. Procédé selon une ou plusieurs des revendications 1 à 8, caractérisé en ce que lors du traitement, le pH est ajusté entre 0 et 6, de préférence entre 1 et 5, en particulier entre 2 et 4.

10. Procédé selon une ou plusieurs des revendications 1 à 9, caractérisé en ce que l'on utilise l'acide sulfurique, l'acide chlorhydrique, l'acide phosphorique ou l'acide acétique pour acidifier.
